# FASCICULE DE BREVET EUROPEEN

(11) **EP 1 720 472 B1**
(45) Date de publication et mention de la délivrance du brevet: **14.05.2008**
(21) Numéro de dépôt: 05732859.3
(22) Date de dépôt: 02.03.2005
(51) Int. Cl.: A61B 17/70

(54) **ELEMENT DE LIAISON DYNAMIQUE POUR UN SYSTEME DE FIXATION RACHIDIEN ET SYSTEME DE FIXATION COMPRENANT UN TEL ELEMENT DE LIAISON**
DYNAMISCHES VERBINDUNGSELEMENT FÜR EIN WIRBELSÄULENBEFESTIGUNGSSYSTEM UND WIRBELSÄULENBEFESTIGUNGSSYSTEM MIT DIESEM VERBINDUNGSELEMENT
DYNAMIC LINKING ELEMENT FOR A SPINAL ATTACHMENT SYSTEM, AND SPINAL ATTACHMENT SYSTEM INCLUDING SAID LINKING ELEMENT

(30) Priorité: 02.03.2004 FR 0402150
(43) Date de publication de la demande: 15.11.2006
(73) Titulaire: Spinevision, 75012 Paris (FR)
(72) Inventeur: PETIT, Dominique, F-62180 Verton (FR); DROULOUT, Thomas, F-78400 Chatou (FR)
(74) Mandataire: Bredema
(86) Numéro de dépôt international: PCT/FR2005/000496
(87) Numéro de publication internationale: WO 2005/087121

(56) Documents cités:
- EP-A- 0 669 109
- FR-A- 2 799 949
- US-A- 5 704 936
- US-A- 5 725 582
- US-A1- 2003 023 241

## Description

La présente invention se rapporte au domaine des systèmes de fixation rachidiens pour la liaison de vertèbres entre elles, et plus particulièrement au domaine des éléments de liaison destinés à maintenir un espacement entre au moins deux éléments d'ancrage implantés respectivement dans une vertèbre.

Il existe actuellement deux types de liaisons rachidiennes : d'une part les liaisons d'ostéosynthèse, et d'autre part les liaisons dynamiques.

Les liaisons d'ostéosynthèse rachidiennes sont des liaisons bien connues. Elles sont en effet couramment utilisées pour consolider plusieurs vertèbres consécutives. Elles ont pour but de figer les vertèbres liées dans une configuration particulière et de stabiliser celles-ci pendant la fusion osseuse afin de permettre une stabilisation fixe dans la situation figée. De telles liaisons consistent en des tiges rigides.

A contrario, les liaisons dynamiques sont utilisées pour réduire les contraintes sur les facettes articulaires et sur les disques intervertébraux en autorisant certains mouvements, tout en réalignant, si nécessaire, les vertèbres les unes par rapport aux autres.

L'art antérieur connaît déjà de tels éléments de liaison.

En particulier, il est proposé dans la demande de brevet européen EP0669109 un dispositif de stabilisation de vertèbres dorsales avoisinantes. Ledit dispositif comporte un élément de liaison constitué d'une bande réalisée en matière synthétique élastique et présentant une section transversale ronde. Cette bande est destinée à être fixée entre au moins deux vis pédiculaires constituées respectivement d'une tête munie d'un perçage transversal. La fixation de ladite bande sur lesdites vis pédiculaires est réalisée en insérant à travers du perçage transversal ladite bande, laquelle est ensuite fixée sur chacune desdites vis pédiculaire au moyen d'une vis de serrage disposée selon l'axe de la vis correspondante, soit transversalement au perçage. Ledit dispositif comporte en outre un élément d'appui monté autour de ladite bande afin de former un corps résistant à la pression.

Un tel élément de liaison présente cependant l'inconvénient de n'effectuer aucun rappel en torsion afin de s'opposer à des mouvements de pivotement des vertèbres autour des disques.

Un autre inconvénient important de cet élément de liaison est qu'il ne peut pas être cintré de façon à s'adapter à la lordose naturelle du rachis lombaire.

En outre, un autre inconvénient est que l'élément de liaison occupe un volume important (de l'ordre de 12,5 millimètres). Dans certaines circonstances, il peut s'avérer difficile d'empêcher que l'élément de liaison en question ne rentre en contact avec des os, un tel contact provoquant de fortes douleurs.

Par ailleurs, un tel dispositif présente un inconvénient particulièrement important lié au fait de la nécessité de choisir la longueur de l'élément d'appui avant la mise en place de ladite bande. Il peut arriver cependant que la distance effective entre les vis après mise en tension de la bande ne soit pas exactement celle souhaitée. Or, le dispositif tel que configuré ne permet aucune liberté de distraction et/ou de compression entre les vis après la mise en place de la bande et de l'élément d'appui. Le chirurgien n'a donc d'autre choix que de démonter l'ensemble élément d'appui et bande pour introduire un nouvel élément d'appui présentant une longueur différente.

Il est également proposé, dans la demande de brevet internationale WO02/07621, une pièce de liaison destinée à maintenir un espacement entre au moins deux éléments d'ancrage vissés dans des vertèbres, ladite pièce de liaison comprenant : i) une partie flexible divisée en deux branches continues espacées l'une de l'autre, lesdites branches étant sensiblement symétriques par rapport à l'axe longitudinal de ladite pièce, les extrémités desdites branches étant reliées entre elles deux à deux et définissant un premier plan moyen, et ii) deux parties rigides formant tiges, présentant une première portion de fixation et une deuxième portion, chaque dite deuxième portion desdites deux parties rigides prolongeant respectivement dans des directions opposées lesdites extrémités desdites branches reliées entre elles deux à deux, la section droite de chacune desdites branches étant inférieure à la section droite desdites parties rigides de façon que ladite pièce de liaison, dont lesdites portions de fixation sont fixées respectivement sur chacun des deux éléments d'ancrage, soit apte à fléchir élastiquement perpendiculairement audit plan moyen lors du déplacement relatif des vertèbres, par quoi les vertèbres, maintenues espacées l'une de l'autre, sont mobiles l'une par rapport à l'autre.

Cette pièce de liaison présente cependant l'inconvénient de ne pouvoir fléchir que dans une direction bien déterminée, à savoir dans la perpendiculaire au plan moyen que forme les deux branches. Il s'ensuit un montage de l'ensemble du système de stabilisation comprenant de telles pièces de liaison nécessitant une certaine précision et donc pouvant s'avérer fastidieux.

Un autre inconvénient d'une telle pièce de liaison s'avère être également son volume.

La demande de brevet US5704936, étant l'état de la technique le plus proche de l'invention, décrit un élément de liaison pour un système de fixation rachidien, destiné à lier au moins deux ensembles de connexion implantables. L'élément de liaison est constitué par une tige comprenant une partie souple comportant un câble lequel est constitué d'au moins un brin élastique.

Un premier objet de l'invention est de remédier aux inconvénients des éléments de liaison dynamique décrits précédemment en proposant un élément de liaison présentant une amplitude de flexion équivalente au regard de ces éléments de liaison dynamiques, mais dont le maintien en rotation est assuré.

Ledit élément de liaison objet de l'invention a également pour avantage de pouvoir être cintré de façon à s'adapter à la lordose naturelle du rachis lombaire.

Ledit élément de liaison objet de l'invention présente également l'avantage d'être peu invasif, tout en assurant les fonctionnalités exigées pour un élément de liaison dynamique (flexibilité, résistance à l'usure, ...).

Ledit élément de liaison présente encore l'avantage de permettre un montage rapide sur les éléments d'ancrage fixés sur les vertèbres.

Ledit élément de liaison présente enfin l'avantage de pouvoir subir une distraction et/ou une compression après sa mise en place sur les éléments d'ancrage.

Pour ce faire, la présente invention concerne un élément de liaison pour un système de fixation rachidien, destiné à lier au moins deux ensembles de connexion implantables, ledit élément de liaison étant remarquable en ce qu'il est constitué d'un câble et d'une enveloppe en polymère entourant ledit câble, ledit câble étant constitué d'au moins un brin élastique coaxial avec ladite enveloppe de sorte à former l'âme de l'élément de liaison.

Afin d'alléger la suite de la description, ledit brin formant l'âme dudit élément de liaison est défini en tant que brin central.

De préférence, ledit élément de liaison comporte au moins une couche de 6 brins au moins répartis autour dudit brin central.

Selon une configuration avantageuse de l'invention, ledit élément de liaison comporte deux couches de brins successives disposées autour dudit brin central, la première couche de brins entourant ledit brin central étant constituée de 6 brins, la seconde couche de brins entourant ladite première couche étant constituée de 12 brins.

Avantageusement, les brins constituant la (ou les) couche(s) consistent en des brins torsadés autour dudit brin central.

Avantageusement, les brins de la (ou des) couche(s) sont constitués d'un matériau différent de celui dudit brin central.

Avantageusement, le brin central présente un diamètre différent de celui des brins de la (ou desdites) couche(s). Selon le type de configuration souhaitée, il peut être inférieur ou supérieur à celui des brins desdites couches.

Avantageusement, les brins constituant la (ou les) couche(s) sont constitués de titane ou d'inox.

Avantageusement, le brin central est tubulaire.

Avantageusement, le brin central est constitué d'un alliage de Nickel-Titane, de Titane, d'Inox ou de Polymère, comme par exemple de PEEK ou de Polyuréthane.

Avantageusement, ladite enveloppe est en Polyuréthane, en PEEK, ou constituée d'un tissu biocompatible.

Un second objet de l'invention est de proposer un élément de liaison combinant les fonctionnalités d'un élément de liaison dynamique à celles d'un élément de liaison d'ostéosynthèse. Plus particulièrement, ledit élément de liaison a pour objet de proposer, conjointement à une liaison dynamique de deux vertèbres au moins, la liaison rigide d'autres vertèbres.

En effet, dans le cas de la mise en place d'un système de fixation et de stabilisation de vertèbres multi niveaux (instrumentation de plusieurs vertèbres), il peut s'avérer nécessaire de lier certaines vertèbres entre elles au moyen d'une liaison dynamique afin d'autoriser certains mouvements, et au contraire de lier les autres vertèbres de façon à ce qu'aucun mouvement ne soit autorisé durant la fusion osseuse (liaison d'ostéosynthèse). Dans les systèmes de fixation et de stabilisation actuels, les éléments de liaison dynamiques sont reliés aux éléments de liaison rigides au moyen d'éléments de fixation supplémentaires tels que des dominos. L'utilisation de pièces supplémentaires a pour inconvénient d'augmenter le temps de montage des éléments de liaison sur les éléments d'ancrage.

La présente invention se rapporte donc également à un élément de liaison pour un système de fixation rachidien, destiné à lier au moins deux ensembles de connexion implantables, caractérisé en ce qu'il comprend une partie souple prolongée à l'une de ses extrémités au moins par une partie rigide, ladite partie souple comportant un câble entouré au moins en partie par une enveloppe en polymère, ledit câble étant constitué d'au moins un brin élastique coaxial avec ladite enveloppe.

L'élément de liaison ainsi configuré permet d'offrir des « liaisons » dynamiques et rigides de vertèbres sans avoir recours à des éléments de fixation supplémentaires.

Cet élément de liaison est défini dans la suite de la description comme étant un élément de liaison semi-dynamique.

Par ailleurs, et de même que précédemment, afin d'alléger la suite de la description, ledit brin élastique est nommé en « brin central ».

De préférence, ladite partie rigide présente une cavité destinée à recevoir au moins en partie ledit câble, ladite cavité étant borgne ou traversante.

Avantageusement, ladite cavité est configurée pour coopérer étroitement avec ledit câble.

Avantageusement, ladite cavité présente une zone évasée en direction de l'extrémité recevant ledit câble.

Avantageusement, la partie souple est fixée sur ladite partie rigide par collage, sertissage ou soudure.

De préférence, ledit câble comporte, de préférence, au moins une couche de 6 brins au moins, lesdits brins étant répartis autour dudit brin central. Selon une configuration avantageuse de l'invention, ledit câble comporte deux couches de brins successives disposées autour dudit brin central, la première couche de brins entourant ledit brin central étant constituée de 6 brins, la seconde couche de brins entourant ladite première couche étant constituée de 12 brins.

Avantageusement, les brins constituant la (ou les) couche(s) consistent en des brins torsadés autour dudit brin central.

Avantageusement, les brins de la (ou des) couche(s) sont constitués d'un matériau différent de celui dudit brin central.

Avantageusement, le brin central est de diamètre différent de celui des brins de la (ou desdites) couche(s).

Avantageusement, les brins constituant la (ou les) couche(s) sont constitués de titane ou d'inox.

Avantageusement, le brin central est tubulaire.

Avantageusement, le brin central est constitué d'un alliage de Nickel-Titane, de Titane, d'Inox ou de Polymère, comme par exemple du PEEK ou du Polyuréthane.

Avantageusement, ladite enveloppe est en Polyuréthane, en PEEK, ou constituée d'un tissu biocompatible.

La présente invention se rapporte également à un système de fixation rachidien comportant au moins deux ensembles de connexion implantables liés à l'aide au moins d'un ou des deux éléments de liaison précédemment décrits.

On comprendra mieux l'invention à l'aide de la description, faite ci-après à titre purement explicatif, d'un mode de réalisation de l'invention, en référence aux figures annexée :
- la figure 1 illustre une vue latérale en perspective d'un élément de liaison dynamique selon l'invention ;
- la figure 2 illustre une variante de réalisation de l'élément de liaison de la figure 1 ;
- la figure 3 illustre une vue en coupe de l'élément de liaison de la figure 2 ;
- la figure 4 illustre une vue partielle en perspective d'un système de fixation rachidien comprenant des éléments de liaison rigide et des éléments de liaison dynamiques selon l'invention ; et
- la figure 5 illustre une vue en coupe d'un élément de liaison semi-dynamique selon un mode de réalisation préféré de l'invention.

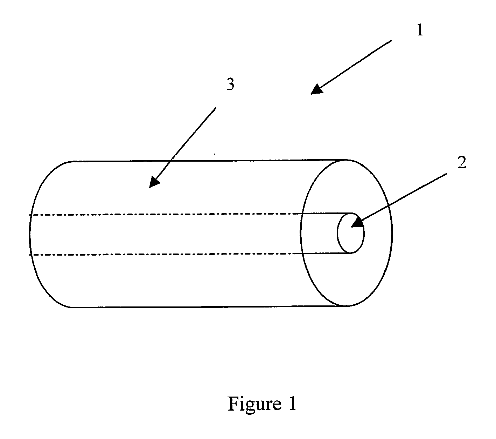

Les éléments de liaison (1), représentés sur les figures 1 à 4, constituent des liaisons dynamiques telles que définies précédemment. Ces éléments de liaison sont destinés à lier au moins deux ensembles de connexion implantables.

L'élément de liaison (1), illustré sur la figure 1, est constitué d'un câble (2) entouré d'une enveloppe (3) relativement souple. Ledit câble (2) consiste, quant à lui, en un brin ou tige élastique.

Par brin, on entend un brin constitué soit d'un seul tenant (« monobrin »), soit de plusieurs fils.

Avantageusement, ledit brin est coaxial avec ladite enveloppe (3) de sorte à constituer l'âme centrale dudit élément de liaison (1).

Dans ce qui suit, ledit câble (2) sera nommé « brin central », et sera référencé également sous le chiffre (2).

Ladite enveloppe (3) est constituée d'un polymère souple, tel que le Polyuréthane ou le PEEK (polyétheréthercétone). Dans une configuration particulière de l'invention, ladite gaine est un tissu biocompatible.

Parallèlement, afin d'offrir le rappel nécessaire pour s'opposer aux mouvements de pivotement des vertèbres autour des disques, ledit câble, lorsqu'il ne comprend qu'un seul brin, est avantageusement constitué en alliage de Titane, en PEEK, ou en alliage super élastique du type alliage Nickel / Titane, également connu sous le nom de Nitinol®.

Afin d'améliorer la caractéristique relative à l'élasticité de l'élément de liaison, une ou plusieurs couches de brins successives sont disposées autour dudit brin central (2).

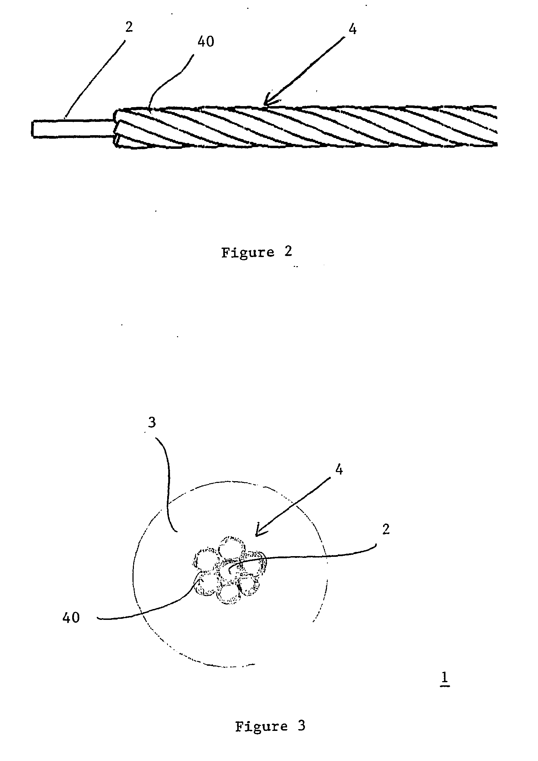

Les figures 2 et 3 illustrent en particulier un élément de liaison (1) comportant une couche (4) de six brins (40) répartis autour dudit brin central (2).

Avantageusement, lesdits brins (40) sont disposés torsadés autour dudit brin central (2).

Selon un autre mode de réalisation préférentiel de l'invention, ledit élément de liaison (1) est caractérisé, en ce qu'il comporte une seconde couche de brins, constituée avantageusement de douze brins, et entourant ladite première couche (4) de six brins (40).

Ces deux configurations de couches sont ici données à titre d'exemple. Il est bien entendu évident pour l'homme du métier que l'organisation et le nombre de couches de brins, ainsi que le nombre de brins par couche et leur configuration, seront fonction de la rigidité (ou élasticité) souhaitée pour ledit élément de liaison (1).

Cependant, le choix de la forme et de la constitution du câble sera guidé par la contrainte du diamètre, le but étant de réaliser un élément de liaison de faible diamètre (de préférence inférieur ou égal à 6 millimètres) de sorte que ledit élément de liaison soit le moins invasif possible.

De même que le brin central (2), les brins de chacune des couches sont en matériaux élastiques. Avantageusement, les brins constituant lesdites couches ainsi que le brin central (2) sont constitués de Titane, d'Inox ou PEEK.

Il est à noter cependant qu'il n'est cependant pas nécessaire que les brins constituant lesdites couches soient réalisées dans le même matériau que celui dans lequel est réalisé ledit brin central (2).

De même, ledit brin central (2) peut présenter également une forme ou des dimensions différentes de celles des brins constituant lesdites couches. Notamment, selon une configuration particulière de l'invention, ledit brin central (2) est constitué d'un tube. Dans ce cas, ledit brin central est de préférence en PEEK, les brins desdites couches étant en Titane ou Inox.

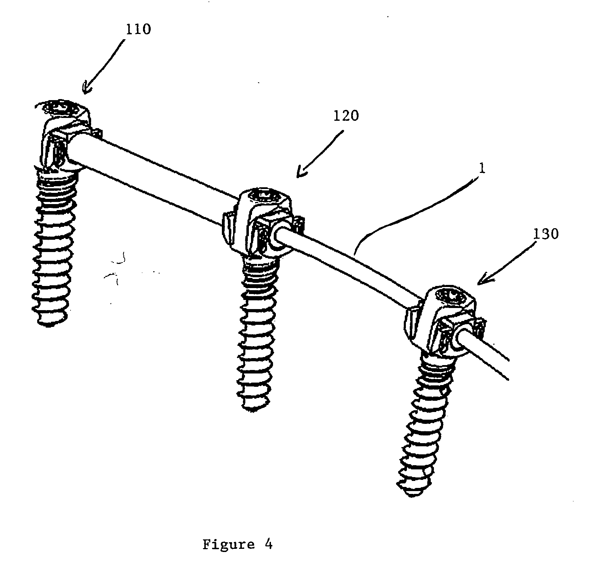

La figure 4 illustre une vue partielle en perspective d'un système de fixation rachidien (100).

Ledit système de fixation comprend une pluralité d'ensembles de connexion implantables. Seulement trois de ces ensembles de connexion implantables sont représentés sur la figure 4, ces trois ensembles de connexion étant respectivement référencés 110, 120, 130.

Chaque ensemble de connexion est respectivement relié à un ensemble de connexion voisin par un élément de liaison. En particulier, dans cet exemple de réalisation, l'ensemble de connexion (110) est relié à l'ensemble de connexion (120) au moyen d'un élément de liaison d'ostéosynthèse rachidienne, l'ensemble de connexion (120) étant relié à l'ensemble de connexion (130) au moyen d'un élément de liaison dynamique selon l'un des modes de réalisation illustrés sur les figures 1 à 3.

La combinaison d'éléments de liaison dynamique et d'éléments de liaison d'ostéosynthèse rachidienne permet ainsi de proposer un système de fixation modulable comprenant des éléments de liaison classique du type liaisons d'ostéosynthèse et des éléments de liaison dynamiques.

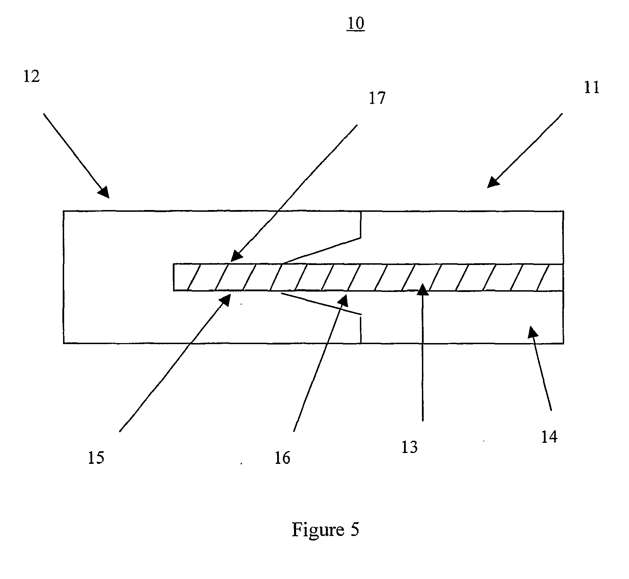

La figure 5 illustre une vue en coupe d'un élément de liaison (10) selon un mode de réalisation préféré de l'invention. Ledit élément de liaison (10) est avantageux en qu'il constitue une liaison « semi-dynamique ».

Ledit élément de liaison (10), en forme de tige, est constitué d'une partie souple (11) et d'une partie rigide (12), ladite partie rigide (12) étant fixée dans le prolongement de ladite partie souple (11). Le comportement « semi-dynamique » dudit élément de liaison (10) est conféré par chacune des parties (11, 12), la partie souple (11) jouant le rôle de liaison dynamique, et la partie rigide (12) le rôle de liaison d'ostéosynthèse.

Avantageusement, ladite partie souple (11) est constituée d'un câble (13) entouré au moins en partie par une enveloppe en polymère (14), ledit câble (13) étant constitué d'au moins un brin élastique coaxial avec ladite enveloppe (14). Ledit câble (13) présente au niveau d'une de ses extrémités une zone dénudée (17) de ladite enveloppe (14).

La partie rigide (12) présente une cavité (15) borgne dans laquelle vient se loger la zone dénudée (17) dudit câble (13). Avantageusement, ladite cavité (15) est configurée pour permettre une coopération étroite avec ledit câble (13).

De par sa constitution et sa fonction, ladite partie souple (11), et donc ledit câble, sont régulièrement soumis à des oscillations. Or, un tel mouvement génère un risque de cisaillement dudit câble (13). En effet, ledit câble (13) est fléchi contre les arêtes coupantes formées par les parois latérales de ladite cavité (15) et la face constituant l'extrémité de la partie rigide (12). Aussi, et afin de limiter ce risque de cisaillement, ladite cavité (15) présente sur l'extrémité débouchante, une zone évasée (16).

Le principe pour réaliser ledit élément de liaison (10) est comme suit.

Ladite cavité (15) borgne est formée longitudinalement dans la partie rigide (12) par perçage. Le câble (13) est alors introduit dans ladite cavité (15) jusqu'à atteindre l'extrémité fermée de ladite cavité (15). La partie du câble (13) insérée dans la cavité (15) est fixée dans celle-ci par collage ou sertissage. Une fois le câble (13) disposé et fixé dans la cavité (15) de la partie rigide (12), l'étape finale consiste à former l'enveloppe (14) en injectant un polymère autour de la partie du câble (13) non insérée dans la cavité (15).

Avantageusement, ledit élément de liaison (10) est réalisé de sorte que ledit câble (13) soit coaxial avec la partie rigide (12).

De même que dans les exemples précédemment décrits, ledit câble (13) est constitué soit d'un seul brin élastique, soit d'un brin élastique entouré d'une ou plusieurs couches successives de brins, lesdits brins desdites couches étant avantageusement torsadés.

Par ailleurs, la description faite précédemment relative à la constitution et la configuration du brin central et des brins des couches s'applique également dans le cadre de cette configuration.

D'autre part, il est bien entendu évident que l'élément de liaison (10) semi-dynamique ne se limite pas à la configuration illustrée sur la figure 5. En effet, il est bien entendu évident que la partie souple pourra avantageusement être prolongée de part et d'autre par une partie rigide.

De même, dans le cas d'un système de stabilisation et de fixation de vertèbres multi niveaux, la longueur de la partie souple et de la ou des parties rigides sera fonction du type de liaisons souhaitées entre chaque vertèbre adjacente.

Enfin, l'élément de liaison dynamique pourra avantageusement être formé d'une pluralité de parties souples séparées les unes des autres par une partie rigide.

L'invention est décrite dans ce qui précède à titre d'exemple. Il est entendu que l'homme du métier est à même de réaliser différentes variantes de l'invention sans pour autant sortir du cadre du brevet.

## Revendications

1. Elément de liaison (10) pour un système de fixation rachidien, destiné à lier au moins deux ensembles de connexion implantables, l'élément de liaison étant constitué par une tige comprenant une partie souple (11) prolongée à l'une de ses extrémités au moins par une partie rigide (12), ladite partie souple (11) comportant un câble (13) entouré au moins en partie par une enveloppe (14) en polymère, ledit câble étant constitué d'au moins un brin élastique coaxial avec ladite enveloppe (14).

2. Elément de liaison (10) selon la revendication 1, **caractérisé en ce que** ladite partie rigide présente une cavité (15) borgne destinée à recevoir au moins en partie ledit câble (13).

3. Elément de liaison (10) selon la revendication 1, **caractérisé en ce que** ladite partie rigide présente une cavité (15) traversante destinée à recevoir au moins en partie ledit câble (13).

4. Elément de liaison (10) selon la revendication 2 ou la revendication 3, **caractérisé en ce que** ladite cavité (15) est configurée pour coopérer avec ledit câble (13).

5. Elément de liaison (10) selon l'une quelconque des revendications 2 à 4, **caractérisé en ce que** ladite cavité (15) présente une zone évasée (16) en direction de l'extrémité recevant ledit câble (13).

6. Elément de liaison (10) selon l'une quelconque des revendications 1 à 5, **caractérisé en ce que** la partie souple (11) est fixée sur la partie rigide (12) par collage, sertissage ou soudure.

7. Elément de liaison ( 10) selon l'une quelconque des revendications précédentes, **caractérisé en ce que** le câble (13) comporte au moins une couche (4) de 6 brins (40) au moins, lesdits brins étant répartis autour dudit brin central.

8. Elément de liaison (10) selon l'une quelconque des revendications précédentes, **caractérisé en ce que** ledit câble comporte deux couches de brins successives disposées autour dudit brin central, la première couche de brins entourant ledit brin central étant constituée de 6 brins, la seconde couche de brins entourant ladite première couche étant constituée de 12 brins.

9. Elément de liaison (10) selon la revendication 7 ou la revendication 8, **caractérisé en ce que** les brins constituant la (ou les) couche(s) consistent en des brins torsadés autour dudit brin central.

10. Elément de liaison (10) selon l'une quelconque des revendications 7 à 9, **caractérisé en ce que** les brins de la (ou des) couche(s) sont constitués d'un matériau différent de celui dudit brin central.

11. Elément de liaison (10) selon l'une quelconque des revendications 7 à 10, **caractérisé en ce que** le brin central est de diamètre différent de celui des brins de la (ou desdites) couche(s).

12. Elément de liaison (10) selon l'une quelconque des revendications 7 à 11, **caractérisé en ce que** les brins constituant la (ou les) couche(s) sont constitués de Titane ou d'Inox, d'alliage Titane-Nickel.

13. Elément de liaison (10) selon l'une quelconque des revendications 1 à 12, **caractérisé en ce que** le brin central est tubulaire.

14. Elément de liaison (10) selon l'une quelconque des revendications 1 à 13, **caractérisé en ce que** le brin central est constitué d'un alliage de Nickel-Titane, de Titane, d'Inox ou de Polymère.

15. Elément de liaison (10) selon la revendication précédente, **caractérisé en ce que** le brin central est en PEEK ou en Polyuréthane.

16. Elément de liaison (10) selon l'une quelconque des revendications 1 à 15, **caractérisé en ce que** ladite enveloppe (14) est en Polyuréthane.

17. Elément de liaison (10) selon l'une quelconque des revendications 1 à 15, **caractérisé en ce que** ladite enveloppe (14) est en PEEK.

18. Elément de liaison (110) selon l'une quelconque des revendications 1 à 15, **caractérisé en ce que** ladite enveloppe (14) est constituée d'un tissu biocompatible.

19. Système de fixation rachidien comportant au moins deux ensembles de connexion implantables liés au moyen au moins d'un élément de liaison (10) selon l'une quelconque des revendications 1 à 18.

## Claims

1. A linking element (10) for a spinal fixing system, designed to link at least two implantable connecting assemblies, the linking element being composed of a rod comprising a flexible part (11) extended, at one of its ends, by at least a rigid part (12), said flexible part (11) comprising a cable (13), at least partly surrounded by a polymer casing (14), said cable being composed of at least one resilient strand coaxial with said casing (14).

2. A linking element (10) according to claim 1, **characterized in that** said rigid part has a blind cavity (15) designed to receive at least partly said cable (13).

3. A linking element (10) according to claim 1, **characterized in that** said rigid part has a through cavity (15) designed to receive at least partly said cable (13).

4. A linking element (10) according to claim 2 or claim 3, **characterized in that** said cavity (15) is configured to cooperate with said cable (13).

5. A linking element (10) according to any one of claims 2 to 4, **characterized in that** said cavity (15) has an area flaring (16) towards the end receiving said cable (13).

6. A linking element (10) according to any one of claims 1 to 5, **characterized in that** the flexible part (11) is fixed on the rigid part (12) by gluing, crimping or welding.

7. A linking element (10) according to any one of the preceding claims, **characterized in that** the cable (13) comprises at least a layer (4) of at least 6 strands (40), said strands being distributed around said core strand.

8. A linking element (10) according to any one of the preceding claims, **characterized in that** said cable includes two successive layers of strands positioned around said core strand, the first layer of strands surrounding said core strand being composed of 6 strands, the second layer of strands surrounding said first layer being composed of 12 strands.

9. A linking element (10) according to claim 7 or claim 8, **characterized in that** the strands composing the layer or layers consist of strands twisted about said core strand.

10. A linking element (10) according to any one of claims 7 to 9, **characterized in that** the strands of the layer or layers are made of a material distinct from that of said core strand.

11. A linking element (10) according to any one of claims 7 to 10, **characterized in that** the core strand has a diameter distinct from that of the strands of said layer or layers.

12. A linking element (10) according to any one of claims 7 to 11, **characterized in that** the strands composing the layer or layers are made of titanium or stainless steel, or of a titanium-nickel alloy.

13. A linking element (10) according to any one of claims 1 to 12, **characterized in that** the core strand has a tubular shape.

14. A linking element (10) according to any one of claims 1 to 13, **characterized in that** the core strand is made of a nickel-titanium alloy, of titanium, stainless steel or of a polymer.

15. A linking element (10) according to the preceding claim, **characterized in that** the core is made of PEEK or of polyurethane.

16. A linking element (10) according to any one of claims 1 to 15, **characterized in that** said casing (14) is made of polyurethane.

17. A linking element (10) according to any one of claims 1 to 15, **characterized in that** said casing (14) is made of PEEK.

18. A linking element (10) according to any one of claims 1 to 15, **characterized in that** said casing (14) is made of a biocompatible fabric.

19. A spinal fixing system comprising at least two implantable connecting assemblies linked by at least one linking element (10) according to any one of claims 1 to 18.

## Patentansprüche

1. Verbindungselement (10) für ein Wirbelbefestigungssystem, bestimmt für die Verbindung von mindestens zwei implantierbaren Anschlusseinheiten, wobei das Verbindungselement aus einer Stange besteht, die einen flexiblen Teil (11) umfaßt, der an einem seiner Enden von mindestens einem starren Teil (12) verlängert ist, wobei der genannte flexible Teil (11) ein Kabel (13) aufweist, das mindestens teilweise von einem Polymermantel (14) umgeben ist, wobei das genannte Kabel aus mindestens einer elastischen Schnur besteht, die koaxial zum genannten Mantel (14) verläuft.

2. Verbindungselement (10) gemäß Anspruch 1, **dadurch gekennzeichnet, dass** der genannte starre Teil einen blinden Hohlraum (15) aufweist für die mindestens teilweise Aufnahme des genannten Kabels (13).

3. Verbindungselement (10) gemäß Anspruch 1, **dadurch gekennzeichnet, dass** der genannte starre Teil einen durchgehenden Hohlraum (15) aufweist für die mindestens teilweise Aufnahme des genannten Kabels (13).

4. Verbindungselement (10 gemäß Anspruch 2 oder Anspruch 3, **dadurch gekennzeichnet, dass** der genannte Hohlraum (15) ausgebildet ist, um mit dem genannten Kabel (13) zusammenzuwirken.

5. Verbindungselement (10) gemäß einem der Ansprüche 2 bis 4, **dadurch gekennzeichnet, dass** der genannte Hohlraum (15) eine in Richtung des das genannte Kabel (13) aufnehmenden Endes aufgeweitete Zone (16) aufweist.

6. Verbindungselement (10) gemäß einem der Ansprüche 1 bis 5, **dadurch gekennzeichnet, dass** der flexible Teil (11) an dem statten Teil (12) durch Kleben, Crimpen oder Schweißen befestigt ist.

7. Verbindungselement (10) gemäß einem der vorangehenden Ansprüche, **dadurch gekennzeichnet, dass** das Kabel (13) mindestens eine Schicht (4) aus mindestens 6 Schnüren (40) umfaßt, wobei die genannten Schnüre um die genannte mittlere Schnur herum verteilt sind.

8. Verbindungselement (10) gemäß einem der vorangehenden Ansprüche, **dadurch gekennzeichnet, dass** das genannte Kabel zwei aufeinanderfolgende Schurschichten umfaßt, die um die genannte mittlere Schnur herum angeordnet sind, wobei die erste, die genannte mittlere Schnur umgebende Schnurschicht aus 6 Schnüren besteht, und die zweite, die genannte erste Schicht umgebende Schnurschicht aus 12 Schnüren besteht.

9. Befestigungselement (10) gemäß Anspruch 7 oder Anspruch 8, **dadurch gekennzeichnet, dass** die die Schicht(en) bildenden Schnüre aus um die genannte mittlere Schnur gedrehten Schnüren besteht.

10. Verbindungselement (10) gemäß einem der Ansprüche 7 bis 9, **dadurch gekennzeichnet, dass** die Schüre der Schicht(en) aus einem anderen Material bestehen als das der genannten mittleren Schnur.

11. Verbindungselement (10) gemäß einem der Ansprüche 7 bis 10, **dadurch gekennzeichnet, dass** die mittlere Schnur einen anderen Durchmesser hat als derjenige der Schnüre der genannten Schicht(en).

12. Verbindungselement (10) gemäß einem der Ansprüche 7 bis 11, **dadurch gekennzeichnet, dass** die die Schicht(en) bildenden Schnüre aus Titan oder rostfreiem Stahl oder aus Titan-Nickel-Legierung bestehen.

13. Verbindungselement (10) gemäß einem der Ansprüche 1 bis 12, **dadurch gekennzeichnet, dass** die mittlere Schnur rohrförmig ist.

14. Verbindungselement (10) gemäß einem der Ansprüche 1 bis 13, **dadurch gekennzeichnet, dass** die mittlere Schnur aus einer Nickel-Titan-Legierung, aus Titan, aus rostfreiem Stahl oder aus Polymer besteht.

15. Verbindungselement (10) gemäß dem vorangehenden Anspruch, **dadurch gekennzeichnet, dass** die mittlere Schnur aus PEEK oder Polyurethan besteht.

16. Verbindungselement (10) gemäß einem der Ansprüche 1 bis 15, **dadurch gekennzeichnet, dass** der genannte Mantel (14) aus Polyurethan besteht.

17. Verbindungselement (10) gemäß einem der Ansprüche 1 bis 15, **dadurch gekennzeichnet, dass** der genannte Mantel (14) aus PEEK besteht.

18. Verbindungselement (10) gemäß einem der Ansprüche 1 bis 15, **dadurch gekennzeichnet, dass** der genannte Mantel (14) aus einem biokompatiblen Stoff besteht.

19. Wirbelbefestigungssystem mit mindestens zwei implantierbaren Anschlusseinheiten, die mittels mindestens einem Verbindungselement (10) gemäß einem der Ansprüche 1 bis 18 verbunden sind.
